# EUROPÄISCHE PATENTANMELDUNG

(11) **EP 2 438 906 A2**
(43) Veröffentlichungstag der Anmeldung: **11.04.2012**
(21) Anmeldenummer: 11169388.3
(22) Anmeldetag: 10.06.2011
(51) Int. Cl.: A61K 8/41, A61K 8/46, A61K 8/81, A61Q 5/02, A61Q 5/06

(54) **Mattierungsadditiv für Blondierungen**

(30) Priorität: 17.08.2010 DE 102010039380
(71) Anmelder: Henkel AG & Co. KGaA, 40589 Düsseldorf (DE)
(72) Erfinder: Janßen, Frank, 41470 Neuss (DE); Adomat, Christel, 40591 Düsseldorf (DE); Weser, Gabriele, 41472 Neuss (DE)

(57) **Zusammenfassung**

Die vorliegende Anmeldung betrifft Mittel zur Behandlung von keratinischen Fasern, welches in einem kosmetisch akzeptablen Träger mindestens einen direktziehenden Farbstoff und/oder mindestens ein Pigment, jeweils im blauen bis violetten Farbbereich, und mindestens ein Tensid enthält, wobei das Mittel zusätzlich mindestens ein Copolymer enthält, welches sich von mindestens einem Monomer ableitet, das mindestens eine C-C-Doppelbindung sowie mindestens eine Funktionalität, ausgewählt aus einer Carbonsäureanhydrid-Gruppe oder einer Carbonsäurevinylester-Gruppe, enthält.

## Beschreibung

Gegenstand der vorliegenden Anmeldung sind Mittel zur Behandlung von keratinischen Fasern, insbesondere menschlichen Haaren, die bereits teilweise oder vollständig ergraut sind. Weiterhin werden eine Verwendung zur Haarbehandlung bei Kompensation von unerwünschten Gelbeindrücken des Haares durch die Ergrauung und ein Verfahren zur Haarbehandlung unter Einsatz des erfindungsgemäßen Mittels beschrieben.

Das optische Erscheinungsbild gewinnt immer mehr an Bedeutung. Das Kopfhaar spielt dabei eine große Rolle. Mit zunehmendem Alter unterliegt das Haar einigen Veränderungen, dazu gehört beispielsweise die Haarergrauung. Doch auch junge Menschen können vom Ergrauen der Haare betroffen sein. Graue oder weiße Haare entstehen mit zunehmenden Lebensjahren durch einen ganz natürlichen Alterungsprozess, bei dem weniger Farbpigmente gebildet werden.

Die natürliche Haarfarbe wird durch Melamine in der Cortex der Haarfaser bestimmt, wobei das Verhältnis von der zwei Pigmentklassen, Eumelanine mit bräunlich-schwarzen Tönen und Pheomelanine mit rötlich-orangen Tönen, die eigentliche Haarfarbe bestimmt. Bei der Ergrauung kommt es zum Abbau dieser Pigmente. Auch aufgrund der unterschiedlichen Abbaurate der verschiedenen Melaninpigmentklassen verlieren Haare beim Ergrauen jedoch nicht gleichmäßig ihre färbenden Pigmente. Je nach Ausgangshaarfarbe, insbesondere jedoch bei dunkleren Fasern mit hohem Melaningehalt, verbleibt beim Ergrauen zumeist ein bestimmter Anteil an Farbstoffen, der sich häufig in gelblichen Eindrücken niederschlägt.

Dieser Gelbeindruck von ergrauten Haaren ist jedoch bei den Betroffenen in der Regel unerwünscht. Daher ist man bemüht, diesem Farbeindruck durch den Einsatz von Mitteln, die eine farbgebende Komponente mit der entsprechenden Komplementärfarbe gemäß der Farbenlehre enthalten, entgegenzuwirken.

Die Schwierigkeit bei der Komplementärfärbung besteht darin, die notwendige Menge an farbgebenden Verbindungen auf dem Haar aufzubringen, ohne dabei zuviel Farbstoffe aufzubringen und somit einen ebenfalls unerwünschten blauen oder violetten Farbeindruck zu erzielen.

Aufgabe der vorliegenden Erfindung war daher die Bereitstellung von Mitteln zum Ausgleich von Gelbeindrücken auf ergrauten Haaren, die es erlauben, die aufgebrachte Menge der Farbstoffe oder Pigmente auf dem Haar zu steuern. Dazu ist es notwendig, den Farbaufzug der Pigmente zu verbessern, um somit eine Überdosierung zu vermeiden.

Überraschenderweise wurde gefunden, dass durch Zusatz von bestimmten Polymeren in Rinseoff-Zubereitungen, wie Shampoos, Spülungen oder Haarkuren, der Farbaufzug von Pigmenten und Farbstoffen verbessert wird.

Als geeignete Polymere haben beispielsweise Methylvinylether-Maleinsäureanhydrid-Copolymere, die aus der WO2008014275 A1 für die Zahnpflege bekannt sind, und Vinylpyrrolidinon-VinylacetatCopolymere, die aus der EP545209 A1 für versprühbare Haarstylingmittel offenbart wurden, herausgestellt. Keine der Offenbarungen lehrt das verbesserte Aufbringen von Farbstoffen oder Pigmenten auf keratinische Fasern.

Ein erster Gegenstand der vorliegenden Erfindung ist daher ein Mittel zur Behandlung von keratinischen Fasern, welches in einem kosmetisch akzeptablen Träger mindestens einen direktziehenden Farbstoff und/oder mindestens ein Pigment, jeweils im blauen bis violetten Farbbereich, und mindestens ein Tensid enthält, welches dadurch gekennzeichnet ist, dass das Mittel zusätzlich mindestens ein Copolymer enthält, welches sich von mindestens einem Monomer ableitet, das mindestens eine C-C-Doppelbindung sowie mindestens eine Funktionalität, ausgewählt aus einer Carbonsäureanhydrid-Gruppe oder einer Carbonsäurevinylester-Gruppe, enthält.

Unter keratinhaltigen bzw. keratinischen Fasern werden erfindungsgemäß Pelze, Wolle, Federn und insbesondere menschliche Haare verstanden. Obwohl die erfindungsgemäßen Mittel in erster Linie zum Färben und/oder Aufhellen von keratinhaltigen Fasern geeignet sind, steht prinzipiell einer Verwendung auch auf anderen Gebieten nichts entgegen.

Die erfindungsgemäßen Mittel enthalten die Wirkstoffe in einem kosmetisch akzeptablen Träger. Dieser kosmetische Träger ist im Sinne der Erfindung ist wässrig, alkoholisch oder wässrigalkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als ersten zwingenden Bestandteil enthält das erfindungsgemäße Mittel mindestens einen direktziehenden Farbstoff und/oder mindestens ein Pigment, jeweils im blauen bis violetten Farbbereich. Als blaue oder violette Farbstoffe oder Pigmente werden erfindungsgemäß solche Verbindungen bezeichnet, die in wässriger oder wässrig-alkoholischer Lösung oder Dispersion optisch für den Beobachter einen blauen oder violetten Farbeindruck erzeugen.

Als Pigment oder Farbstoff eignen sich daher solche Verbindungen, die in wässriger oder wässrig-alkoholischer Lösung oder Dispersion einem Chromophor enthalten, dessen Absorptionsmaximum im VIS-Wellenlängenbereich von λ = 380 nm bis 490 nm liegt. Dieses kann der Fachmann durch gängige UV/VIS-Spektroskopiemethoden ermitteln.

Geeignete Farbstoffe oder Pigmente in blauen bis violetten Farbbereich sind insbesondere die Verbindungen, die unter den INCI-Bezeichnungen Pigment Blue 15 (CAS-Nr. 147-14-8), HC Blue No. 2 (CAS-Nr. 33229-34-4), HC Blue No. 11 (CAS-Nr. 23920-15-2), HC Blue No. 12 (CAS-Nr. 132885-85-9), HC Blue No. 14 (CAS-Nr. 99788-75-7), HC Blue No. 15 (CAS-Nr. 74578-10-2), HC Blue No. 16 (CAS-Nr. 502453-61-4), Acid Blue 9 (CAS-Nr. 3844-45-9), Acid Blue 62 (CAS-Nr. 4368-56-3), Basic Blue 99 (CAS-Nr. 68123-13-7), Disperse Blue 377 (CAS-Nr. 67674-26-4), Tetrabromphenolblau (CAS-Nr. 4430-25-5), Acid Violet 43 (auch als Ext. D&C Violet No. 2 bezeichnet; CAS-Nr. 4430-18-6), Disperse Violet 1 (CAS-Nr. 128-95-0), Basic Violet 2 (CAS-Nr. 3248-91-7), HC Violet No. 1 (CAS-Nr. 82576-75-8), HC Violet No. 2 (CAS-Nr. 104226-19-9) und D&C Violet No. 2 (CAS-Nr. 81-48-1) bekannt sind.

Eine Ausführungsform des ersten Erfindungsgegenstands ist daher ein Mittel, welches dadurch gekennzeichnet ist, dass es als direktziehenden Farbstoff und/oder Pigment, jeweils im blauen bis violetten Farbbereich, mindestens eine Verbindung, ausgewählt aus der Gruppe, die gebildet wird aus Pigment Blue 15, HC Blue No. 2, HC Blue No. 11, HC Blue No. 12, HC Blue No. 14, HC Blue No. 15, HC Blue No. 16, Acid Blue 9, Acid Blue 62, Basic Blue 99, Disperse Blue 377, Tetrabromphenolblau, Disperse Violet 1, Acid Violet 43, Basic Violet 2, HC Violet No. 1, HC Violet No. 2 und D&C Violet No. 2, enthält.

Ein bevorzugter Farbstoff oder ein bevorzugtes Pigment, jeweils im blauen bis violetten Farbbereich, ist dabei ausgewählt aus Pigment Blue 15, HC Blue No. 2, HC Blue No. 12, HC Blue No. 16, Acid Violet 43 und D&C Violet No. 2.

Die Farbstoffe oder Pigmente in blauen bis violetten Farbbereich werden bevorzugt in einem Anteil von 0,0001 bis 10,0 Gew.-%, bevorzugt 0,01 bis 5 Gew.-%, besonders bevorzugt 0,05 bis 1,5 Gew.-%, bezogen auf das Gesamtgewicht des anwendungsbereiten Mittels, eingesetzt.

Als weiteren wesentlichen Inhaltsstoff enthält das erfindungsgemäße Mittel weiterhin mindestens ein Tensid. Als Tensid werden oberflächenaktive Substanzen bezeichnet und lassen sich in anionische, kationische, zwitterionischen, amphotere und nichtionische Tensiden unterteilen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether-und Amidgruppen sowie Hydroxylgruppen enthalten sein. Beispiele für geeignete anionische Tenside sind, jeweils in Form der Natrium-, Kalium- und Ammonium- sowie der Mono, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel RO(CH₂CH₂O)ₓCH₂COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare α-Olefinsulfonate mit 8 bis 24 C-Atomen,
- Sulfonate ungesättigter Fettsäuren mit 8 bis 24 C-Atomen und 1 bis 6 Doppelbindungen,
- α-Sulfofettsäuremethylester von Fettsäuren mit 8 bis 30 C-Atomen,
- Alkylsulfate und Alkylethersulfate der Formel RO(CH₂CH₂0)ₓSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel

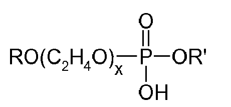

in der R bevorzugt für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht,
- sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht,
- Monoglyceridsulfate und Monoglyceridethersulfate der Formel (MGS) 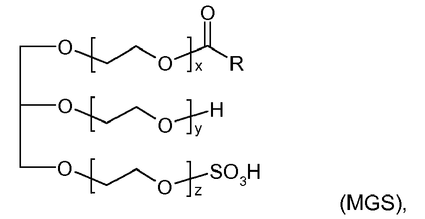 in der R für einen linearen oder verzweigten Alkylrest mit 6 bis 22 Kohlenstoffatomen, und x, y und z in Summe für 0 oder für Zahlen von 1 bis 30, vorzugsweise 2 bis 10 stehen. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vor-zugsweise werden Monoglyceridsulfate der Formel (MGS) eingesetzt, in der R für einen linearen Alkylrest mit 8 bis 18 Kohlenstoffatomen steht.

Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat-oder Sulfat-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das C₁₂-C₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 4 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂-C₆-Alkylrest endgruppenverschlossene Anlagerungsprodukte von 4 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- Polyglycerinester und alkoxylierte Polyglycerinester, wie beispielsweise Poly(3)glycerin-diisostearat (Handelsprodukt: Lameform^{®}TGI (Henkel)) und Poly(2)glycerinpolyhydroxy-stearat (Handelsprodukt: Dehymuls^{®}PGPH (Henkel)).
- Polyolfettsäureester, wie beispielsweise das Handelsprodukt Hydagen^{®} HSP (Cognis) oder Sovermol-Typen (Cognis),
- höher alkoxylierte, bevorzugt propoxylierte und insbesondere ethoxylierte, Mono-, Di- und Triglyceride mit Alkoxylierungsgrad von größer als 5, wie beispielsweise Glycerinmonolaurat + 20 Ethylenoxid, und Glycerinmonostearat + 20 Ethylenoxid,
- Sorbitanfettsäureester und Anlagerungeprodukte von Ethylenoxid an Sorbitanfettsäureester wie beispielsweise die Polysorbate und Sorbitanmonolaurat + 20 Mol Ethylenoxid (EO),
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylphenole und Alkylphenolalkoxylate mit 6 bis 21, insbesondere 6 bis 15 Kohlenstoffatomen in der Alkylkette und 5 bis 30 Ethylenoxid- und/oder Propylenoxid-Einheiten. Bevorzugte Vertreter dieser Klasse sind beispielsweise Nonylphenol + 9 EO und Octylphenol + 8 EO;
- Alkylpolyglykoside entsprechend der allgemeinen Formel RO-(Z)ₓ, wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten.

Als nichtionische Tenside eignen sich insbesondere C₈-C₂₂-Alkylmono- und oligoglycoside und deren ethoxylierte Analoga. Insbesondere die nichtethoxylierten Verbindungen haben sich als besonders geeignet erwiesen.

Besonders bevorzugt sind solche Alkylpolyglykoside der Formel RO-(Z)ₓ, bei denen R
- im Wesentlichen aus C₈- und C₁₀-Alkylgruppen,
- im Wesentlichen aus C₁₂- und C₁₄-Alkylgruppen,
- im Wesentlichen aus C₈- bis C₁₆-Alkylgruppen oder
- im Wesentlichen aus C₁₂- bis C₁₆-Alkylgruppen oder
- im Wesentlichen aus C₁₆- bis C₁₈-Alkylgruppen besteht.

Diese Verbindungen sind dadurch gekennzeichnet, dass als Zuckerbaustein Z beliebige Mono-oder Oligosaccharide eingesetzt werden können. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, dass die gewählten Substanzen bevorzugt frei von Lösungsmitteln, Stellmitteln und anderen Begleitstoffen sein sollen.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die anionischen, nichtionischen, zwitterionischen oder amphoteren Tenside werden in Mengen von 0,1 bis 50 Gew.%, bevorzugt 1 bis 30 Gew.% und ganz besonders bevorzugt von 1 bis 25 Gew.%, bezogen auf die Gesamtmenge des anwendungsbereiten Mittels, eingesetzt.

Erfindungsgemäß bevorzugt sind kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar. Eine erfindungsgemäß besonders geeignete Verbindung der Alkylamidoamine stellt das unter der Bezeichnung Tegoamid^{®} S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform enthalten die Mittel des ersten Erfindungsgegenstands zumindest ein anionisches und/oder ein nichtionisches Tensid. Bevorzugt wird dieses Tensid ausgewählt aus der Gruppe, gebildet aus Alkylsulfaten, Alkylethersulfaten und Ethercarbonsäuren, jeweils mit 10 bis 18 C-Atomen in der Alkylgruppe und bis zu 12 Glykolethergruppen im Molekül, C₈-C₂₂-Alkylmono- und -oligoglycosiden sowie Alkylenoxid-Anlagerungsprodukten an gesättigte lineare Fettalkohole und Fettsäuren mit jeweils 2 bis 30 Mol Ethylenoxid pro Mol Fettalkohol bzw. Fettsäure.

Besonders bevorzugt ist dabei ein Mittel des ersten Erfindungsgegenstands, welches dadurch gekennzeichnet ist, dass das Mittel als Tensid anionische und/oder nichtionische Tenside, bevorzugt in einem Anteil von 1,0 bis 50,0 Gew.-%, bevorzugt 3,0 bis 30 Gew.-%, insbesondere 5,0 bis 25 Gew.-%, jeweils bezogen auf das Gesamtgewicht des anwendungsbereiten Mittels, enthält.

Als dritten wesentlichen Inhaltsstoff enthält das erfindungsgemäße Mittel mindestens ein Copolymer, welches sich von mindestens einem Monomer ableitet, das mindestens eine C-C-Doppelbindung sowie mindestens eine Funktionalität, ausgewählt aus einer Carbonsäureanhydrid-Gruppe oder einer Carbonsäurevinylester-Gruppe, enthält.

Erfindungsgemäße Monomere (M1) mit mindestens einer C-C-Doppelbindung sowie mindestens einer Funktionalität, ausgewählt aus einer Carbonsäureanhydrid-Gruppe oder einer Carbonsäurevinylester-Gruppe, sind ausgewählt aus Maleinsäureanhydrid und/oder Vinylacetat.

Weiterhin leitet sich das Copolymer noch zumindest von einem weiteren Monomer (M2) mit einer C-C-Doppelbindung ab.

Prinzipiell können als Monomer (M2) anionische, kationische oder nichtionische Monomere eingesetzt werden, sofern sie geeignet sind, ein Copolymer mit dem Monomer (M1) auszubilden. Das Copolymer kann dabei ein Blockcopolymer oder ein statistisches Copolymer sein. Erfindungsgemäß bevorzugt sind dabei statistische Copolymere.

Als anionische Monomere (M2) sind im wesentlichen Acrylsäure, Methacrylsäure, Crotonsäure, 2-Acrylamido-2-methylpropansulfonsäure sowie Itaconsäure geeignet.

Als nichtionische Monomere (M2) sind beispielsweise Acrylamid, Methacrylamid, C₁-C₆-Alkyl-Acrylsäureester, C₁-C₆-Alkyl-Methacrylsäureester, Di-(C₁-C₆-Alkyl)acrylamide, Itaconsäuremono- und - diester, Vinylester, Vinylether und Vinyllactame, wie Vinylpyrrolidinon oder Vinylcaprolactam, geeignet.

Als kationische Monomere (M2) sind beispielsweise quaternisierte oder quaternisierbare C₁-C₆-Alkylacrylate und C₁-C₆-Alkylmethacrylate, wie Methacryloxyethyltrimethylammonium Chlorid, gegebenenfalls substituierte, insbesondere quaternisierte oder quaternisierbare Mono-(C₁-C₆-Alkyl)acrylamide, quaternisierte Vinylimidazole, wie 3-Methyl-1-vinylimidazolinium Chlorid, sowie Dimethyldiallylammonium Chlorid geeignet.

Gegebenenfalls sind die erfindungsgemäßen Copolymere vernetzt. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen.

In einer bevorzugten Ausführungsform des ersten Erfindungsgegenstands enthält das Mittel als zusätzliches Copolymer mindestens ein Copolymer mit einem nichtionischen Monomer (M2).

Bevorzugt sind dabei Copolymere von Maleinsäureanhydrid als Monomer (M1) und Alkylvinylethern als Monomer (M2). Ein besonderes bevorzugtes Copolymer ist dabei ein Copolymer aus Methylvinylether und Maleinsäureanhydrid (INCI-Bezeichnung: PVM/MA Copolymer), welches unter dem Handelsnamen Gantrez erhältlich wird. Im wässrigen Milieu werden ehemalige Maleinsäureanhydrid-Einheiten des Polymers zu entsprechenden Maleinsäure-Einheiten hydrolysiert.

Eine bevorzugte Ausführungsform der vorliegenden Erfindung ist daher ein Mittel, welches dadurch gekennzeichnet ist, dass das Mittel als zusätzliches Copolymer mindestens ein Polymerisationsprodukt aus Maleinsäureanhydrid und/oder Maleinsäure als Monomer (M1) sowie Methylvinylether als Monomer (M2) enthält.

Weiterhin bevorzugt sind dabei Copolymere von Vinylacetat als Monomer (M1) und Vinyllactamen, wie Vinylpyrrolidinon und Vinylcaprolactam als Monomer (M2). Ein weiteres bevorzugtes Copolymer ist dabei ein Copolymer aus Vinylpyrrolidinon und Vinylacetat (INCI-Bezeichnung: VP/VA copolymer), welches unter dem Handelsnamen Luviskol erhältlich wird.

Eine bevorzugte Ausführungsform der vorliegenden Erfindung ist daher ein Mittel, welches dadurch gekennzeichnet ist, dass das Mittel das zusätzliche Copolymer in einem Anteil von 0,01 bis 20,0 Gew.-%, bevorzugt 0,05 bis 10 Gew.-%, besonders bevorzugt 0,1 bis 5 Gew.-%, bezogen auf das Gesamtgewicht des anwendungsbereiten Mittels, enthält.

Eine bevorzugte Ausführungsform der vorliegenden Erfindung ist ein Mittel zur Behandlung von keratinischen Fasern, welches in einem kosmetisch akzeptablen Träger mindestens einen direktziehenden Farbstoff und/oder mindestens ein Pigment, ausgewählt aus der Gruppe, die gebildet wird aus Pigment Blue 15, HC Blue No. 2, HC Blue No. 11, HC Blue No. 12, HC Blue No. 14, HC Blue No. 15, HC Blue No. 16, Acid Blue 9, Acid Blue 62, Basic Blue 99, Disperse Blue 377, Tetrabromphenolblau, Acid Violet 43, Disperse Violet 1, Basic Violet 2, HC Violet No. 1, HC Violet No. 2 und D&C Violet No. 2, mindestens ein anionisches und/oder ein nichtionisches Tensid, ausgewählt aus der Gruppe, gebildet aus Alkylsulfaten, Alkylethersulfaten und Ethercarbonsäuren, jeweils mit 10 bis 18 C-Atomen in der Alkylgruppe und bis zu 12 Glykolethergruppen im Molekül, C₈-C₂₂-Alkylmono- und oligoglycosiden sowie Alkylenoxid-Anlagerungsprodukten an gesättigte lineare Fettalkohole und Fettsäuren mit jeweils 2 bis 30 Mol Ethylenoxid pro Mol Fettalkohol bzw. Fettsäure, und mindestens ein Copolymer aus Methylvinylether und Maleinsäureanhydrid enthält.

Eine besonders bevorzugte Ausführungsform der vorliegenden Erfindung ist ein Mittel zur Behandlung von keratinischen Fasern, welches in einem kosmetisch akzeptablen Träger mindestens einen direktziehenden Farbstoff und/oder mindestens ein Pigment, ausgewählt aus der Gruppe, die gebildet wird aus Pigment Blue 15, HC Blue No. 2, HC Blue No. 11, HC Blue No. 12, HC Blue No. 14, HC Blue No. 15, HC Blue No. 16, Acid Blue 9, Acid Blue 62, Basic Blue 99, Disperse Blue 377, Tetrabromphenolblau, Acid Violet 43, Disperse Violet 1, Basic Violet 2, HC Violet No. 1, HC Violet No. 2 und D&C Violet No. 2, als anionisches Tensid mindestens ein Alkylethersulfat mit 10 bis 18 C-Atomen in der Alkylgruppe und bis zu 12 Glykolethergruppen im Molekül, und mindestens ein Copolymer aus Methylvinylether und Maleinsäureanhydrid enthält.

Eine weitere besonders bevorzugte Ausführungsform der vorliegenden Erfindung ist ein Mittel zur Behandlung von keratinischen Fasern, welches in einem kosmetisch akzeptablen Träger mindestens einen direktziehenden Farbstoff und/oder mindestens ein Pigment, ausgewählt aus der Gruppe, die gebildet wird aus Pigment Blue 15, HC Blue No. 2, HC Blue No. 11, HC Blue No. 12, HC Blue No. 14, HC Blue No. 15, HC Blue No. 16, Acid Blue 9, Acid Blue 62, Basic Blue 99, Disperse Blue 377, Tetrabromphenolblau, Acid Violet 43, Disperse Violet 1, Basic Violet 2, HC Violet No. 1, HC Violet No. 2 und D&C Violet No. 2, als anionisches Tensid mindestens eine Ethercarbonsäure mit 10 bis 18 C-Atomen in der Alkylgruppe und bis zu 12 Glykolethergruppen im Molekül, und mindestens ein Copolymer aus Methylvinylether und Maleinsäureanhydrid enthält.

Eine weitere besonders bevorzugte Ausführungsform der vorliegenden Erfindung ist ein Mittel zur Behandlung von keratinischen Fasern, welches in einem kosmetisch akzeptablen Träger mindestens einen direktziehenden Farbstoff und/oder mindestens ein Pigment, ausgewählt aus der Gruppe, die gebildet wird aus Pigment Blue 15, HC Blue No. 2, HC Blue No. 11, HC Blue No. 12, HC Blue No. 14, HC Blue No. 15, HC Blue No. 16, Acid Blue 9, Acid Blue 62, Basic Blue 99, Disperse Blue 377, Tetrabromphenolblau, Acid Violet 43, Disperse Violet 1, Basic Violet 2, HC Violet No. 1, HC Violet No. 2 und D&C Violet No. 2, als nichtionisches Tensid mindestens ein C₈-C₂₂-Alkylmono- und/oder -oligoglycosid, und mindestens ein Copolymer aus Methylvinylether und Maleinsäureanhydrid enthält.

Eine weitere besonders bevorzugte Ausführungsform der vorliegenden Erfindung ist ein Mittel zur Behandlung von keratinischen Fasern, welches in einem kosmetisch akzeptablen Träger mindestens einen direktziehenden Farbstoff und/oder mindestens ein Pigment, ausgewählt aus der Gruppe, die gebildet wird aus Pigment Blue 15, HC Blue No. 2, HC Blue No. 11, HC Blue No. 12, HC Blue No. 14, HC Blue No. 15, HC Blue No. 16, Acid Blue 9, Acid Blue 62, Basic Blue 99, Disperse Blue 377, Tetrabromphenolblau, Acid Violet 43, Disperse Violet 1, Basic Violet 2, HC Violet No. 1, HC Violet No. 2 und D&C Violet No. 2, als nichtionisches Tensid mindestens ein Alkylenoxid-Anlagerungsprodukt an gesättigte lineare Fettalkohole mit 2 bis 30 Mol Ethylenoxid pro Mol Fettalkohol, und mindestens ein Copolymer aus Methylvinylether und Maleinsäureanhydrid enthält.

Durch Zusatz bestimmter Elektrolyte können die erfindungsgemäßen Mittel in ihrer Wirkung weiter verbessert werden. Bevorzugt ist hierbei der Zusatz von anorganischen Elektrolyten, insbesondere Alkalimetall- und/oder Ammoniumchlorid. Besonders bevorzugt sind Mittel, welche zusätzlich Natriumchlorid enthalten.

Eine weitere Ausführungsform der vorliegenden Erfindung ist ein Mittel des ersten Erfindungsgegenstands, welches dadurch gekennzeichnet ist, dass das Mittel zusätzlich mindestens einen anorganischen Elektrolyt, bevorzugt Natriumchlorid, in einem Anteil von 0,1 bis 20 Gew.-%, bevorzugt 0,5 bis 5 Gew.-%, bezogen auf das Gesamtgewicht des anwendungsbereiten Mittels, enthält.

Die anwendungsbereiten Aufhellmittel können weitere Hilfs- und Zusatzstoffe enthalten. So hat es sich als vorteilhaft erwiesen, wenn die Mittel mindestens ein weiteres Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete zusätzliche Verdickungsmittel sind
- nichtionische, synthetische Polymere, wie beispielsweise Vinylpyrrolidinon/Vinylacrylat-Copolymere, Polyvinylpyrrolidinon, Polyethylenglykole und Polysiloxane;
- anionische, synthetische Polymere, wie beispielsweise Polyacrylsäuren oder vernetzte Polyacrylsäuren;
- kationische, synthetische Polymere, wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol;
- natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Ferner können die erfindungsgemäßen Mittel weitere Wirk-, Hilfs- und Zusatzstoffe enthalten, beispielsweise Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide, insbesondere Arginin und/oder Serin; Proteinhydrolysate auf tierischer und/oder pflanzlicher Basis, wie beispielsweise Elastin-, Kollagen-, Keratin-, Seiden- und Milcheiweiß-Proteinhydrolysate, oder Mandel-, Reis-, Erbsen-, Kartoffel-und Weizenproteinhydrolysate, sowie in Form ihrer Fettsäure-Kondensationsprodukte oder gegebenenfalls anionisch oder kationisch modifizierten Derivate; pflanzliche Öle, wie Macadamianussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl; Lichtschutzmittel, wie derivatisierte Benzophenone, Zimtsäure-Derivate und Triazine; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen, insbesondere der Gruppen A, B₃, B₅, B₆, C, E, F und H; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente; Oxidationsmittel, insbsondere Wasserstoffperoxid; Komplexbildner, wie EDTA, HEDP oder deren Salze; pH-Stellmittel, insbesondere Acidifizierungsmittel wie Genuss-Säuren, beispielsweise Zitronensäure, Essigsäure, Äpfelsäure oder Weinsäure, sowie verdünnte Mineralsäuren, und Alkalisierungsmittel wie anorganischen Salzen, insbesondere der Alkali- und Erdalkalimetalle, organischen Alkalisierungsmitteln, insbesondere Aminen, basische Aminosäuren und Alkanolaminen, und Ammoniak; sowie Treibmittel wie Propan-Butan-Gemische, N₂O Dimethylether, CO₂ und Luft

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Die erfindungsgemäßen Mittel besitzen zur Schonung von Haaren und Haut einen nicht zu hohen pH-Wert. Daher ist es bevorzugt, wenn der pH-Wert des Mittels zwischen pH 3 und 9, insbesondere zwischen 4 und 8, liegt. Bei den pH-Werten im Sinne der vorliegenden Erfindung handelt es sich um pH-Werte, die bei einer Temperatur von 22 °C gemessen wurden.

Die erfindungsgemäßen Mittel besitzen bevorzugt eine Viskosität, die es ermöglicht, dass das Mittel sich einerseits gut auftragen lässt und andererseits über ein solches Fließverhalten verfügt, dass es für das Mittel eine ausreichend lange Einwirkzeit am Wirkort garantiert.

Die Viskosität lässt sich nach verschiedenen Standardmethoden bestimmen, bei denen insbesondere Geräteparameter, Messtemperatur Spindelgeometrie und deren Drehgeschwindigkeit Einfluss auf das Messergebnis haben. Dem Fachmann sind diese unterschiedlichen Messmethoden geläufig.

In einer bevorzugten Ausführungsform des ersten Erfindungsgegenstands besitzt das Mittel eine Viskosität von 1000 bis 30000 mPa·s, bevorzugt von 3000 bis 10000 mPa·s, jeweils gemessen mit Haake Messgerät VT 550 bei 20 °C, einer Rotations-Frequenz von 8 min⁻¹ und mit einem Dreh-/ Messsystem MV II.

Die erfindungsgemäßen Mittel werden bevorzugt zur Behandlung von keratinischen Fasern, insbesondere ergrauten menschlichen Haaren, eingesetzt.

Ein zweiter Gegenstand der vorliegenden Erfindung ist daher ein Verfahren zur Behandlung von menschlichen Haaren, welches dadurch gekennzeichnet ist, dass ein Mittel des ersten Erfindungsgegenstands auf menschliches Haar aufgetragen wird, für eine Einwirkdauer von 1 bis 45 min im Har belassen wird und anschließend das Haar mit einer wässrigen Zubereitung ausgespült wird.

Bevorzugt beträgt die Einwirkzeit der anwendungsbereiten Mittel 1 bis 45 min, insbesondere 5 bis 40 min, besonders bevorzugt 10 bis 35 min. Während der Einwirkzeit des Mittels auf der Faser kann es vorteilhaft sein, den Aufhellvorgang durch Wärmezufuhr zu unterstützen. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Die Mittel ergeben bereits bei physiologisch verträglichen Temperaturen von unter 45°C gute Behandlungsergebnisse.

Nach Ende der Einwirkzeit wird das verbleibende Mittel mit einer wässrigen Zubereitung, insbesondere mit Wasser selbst oder einem wässrigen Reinigungsmittel aus dem Haar gespült. Als Reinigungsmittel kann dabei insbesondere ein handelsübliches Shampoo dienen, wobei insbesondere dann auf das Reinigungsmittel verzichtet werden kann und der Ausspülvorgang mit Leitungswasser erfolgen kann, wenn das erfindungsgemäße Mittel einen stark tensid-haltigen Träger besitzt.

Die bevorzugten Ausführungsformen des ersten Erfindungsgegenstands gelten mutatis mutandis auch für den zweiten Erfindungsgegenstand.

Ein dritter Gegenstand der vorliegenden Erfindung ist die kosmetische Verwendung eines Mittels des ersten Erfindungsgegenstands zur Neutralisierung von gelblichen Verfärbungen auf ergrauten menschlichen Haaren.

Die Ausführungsformen des ersten und zweiten Erfindungsgegenstands gelten mutatis mutandis für die Verwendung des dritten Erfindungsgegenstands.

### Beispiele

Es wurden die folgenden Shampoo-Zubereitungen (Mengenangaben in Gewichtsprozent) hergestellt:

| | A | B |
|---|---|---|
| Gantrez S 97 BF | 2,00 | 0,50 |
| HC Blue No. 12 | 0,05 | 0,01 |
| D&C VIOLET No. 2 | 0,20 | 0,10 |
| Salicylsäure | 0,20 | 0,20 |
| Texapon K 14 S | 15,00 | 15,00 |
| PEG-7 Coconut glycerides | 2,00 | 2,00 |
| Natriumbenzoat | 0,50 | 0,50 |
| Disodium Cocoamphodiacetate | 3,50 | 3,50 |
| Euperlan PK 3000 AM | 2,60 | 2,60 |
| Lamesoft PO 65 | 1,00 | 1,00 |
| Benzophenone-4 | 0,40 | 0,40 |
| Arlypon F | 0,40 | 0,40 |
| Antil 141 L | 0,40 | 0,40 |
| Natriumchlorid | 1,00 | 1,00 |
| EDTA, Na₂-Salz | 0,10 | 0,10 |
| Carbomer | 0,20 | 0,20 |
| Polymer JR 400 | 0,40 | 0,40 |
| Wasser, vollentsalzt | ad 100 | ad 100 |

| | | |
|---|---|---|
| Folgende Rohstoffe wurden eingesetzt: Gantrez S 97 BF (INCI-Bezeichnung: PVM/MA Copolymer; ISP); Texapon K 14 S (ca. 79% Gew.-% in Wasser, INCI-Bezeichnung: Sodium Myreth-2 Sulfate; Cognis Deutschland); Euperlan PK 3000 AM (ca. 79% Gew.-% in Wasser, INCI-Bezeichnung: Aqua, Glycol Distearate, Glycerin, Laureth-4, Cocoamidepropyl Betaine, Formic Acid); Lamesoft PO 65 (ca. 66 Gew.-% in Wasser, INCI-Bezeichnung: Coco-Glucoside, Glyceryl Oleate, Aqua; Cognis Deutschland); Arlypon F (INCI-Bezeichnung: Laureth-2; Cognis Deutschland); Antil 141 L (ca. 40 Gew.-% in Wasser, INCI-Bezeichnung: PEG-55 Propylene Glycol Oleate, Propylene Glycol, Aqua; Evonik); Polymer JR 400 (INCI-Bezeichnung: Polyquaternium-10; Amerchol). | | |

Die erfindungsgemäßen Mittel A und B wurden jeweils auf teilweise ergrautem Haar für eine Einwirkzeit von 5 min bei Raumtemperatur angewendet und führten zu einer deutlichen optischen Reduktion der Gelbanteile im Gesamtfarbeindruck des Haares.

## Patentansprüche

1. Mittel zur Behandlung von keratinischen Fasern, welches in einem kosmetisch akzeptablen Träger mindestens einen direktziehenden Farbstoff und/oder mindestens ein Pigment, jeweils im blauen bis violetten Farbbereich, und mindestens ein Tensid enthält,
**dadurch gekennzeichnet, dass** das Mittel zusätzlich mindestens ein Copolymer enthält, welches sich von mindestens einem Monomer ableitet, das mindestens eine C-C-Doppelbindung sowie mindestens eine Funktionalität, ausgewählt aus einer Carbonsäureanhydrid-Gruppe oder einer Carbonsäurevinylester-Gruppe, enthält.

2. Mittel nach Anspruch 1, **dadurch gekennzeichnet, dass** es als direktziehenden Farbstoff und/oder Pigment, jeweils im blauen bis violetten Farbbereich, mindestens eine Verbindung, ausgewählt aus der Gruppe, die gebildet wird aus Pigment Blue 15, HC Blue No. 2, HC Blue No. 11, HC Blue No. 12, HC Blue No. 14, HC Blue No. 15, HC Blue No. 16, Acid Blue 9, Acid Blue 62, Basic Blue 99, Disperse Blue 377, Tetrabromphenolblau, Acid Violet 43, Disperse Violet 1, Basic Violet 2, HC Violet No. 1, HC Violet No. 2 und D&C Violet No. 2.

3. Mittel nach einem der Ansprüche 1 oder 2, **dadurch gekennzeichnet, dass** das Mittel direktziehende Farbstoff und/oder Pigmente, jeweils im blauen bis violetten Farbbereich in einem Anteil von 0,0001 bis 10,0 Gew.-%, bevorzugt 0,01 bis 5 Gew.-%, besonders bevorzugt 0,05 bis 1,5 Gew.-%, bezogen auf das Gesamtgewicht des anwendungsbereiten Mittels, enthält.

4. Mittel nach einem der Ansprüche 1 bis 3, **dadurch gekennzeichnet, dass** das Mittel als Tensid anionische und/oder nichtionische Tenside, bevorzugt in einem Anteil von 1,0 bis 50,0 Gew.-%, bevorzugt 3,0 bis 30 Gew.-%, insbesondere 5,0 bis 25 Gew.-%, jeweils bezogen auf das Gesamtgewicht des anwendungsbereiten Mittels, enthält.

5. Mittel nach einem der Ansprüche 1 bis 4, **dadurch gekennzeichnet, dass** das Mittel als zusätzliches Copolymer mindestens ein Polymerisationsprodukt aus Maleinsäureanhydrid und/oder Maleinsäure als Monomer (M1) sowie Methylvinylether als Monomer (M2) enthält.

6. Mittel nach einem der Ansprüche 1 bis 5, **dadurch gekennzeichnet, dass** das Mittel das zusätzliche Copolymer in einem Anteil von 0,01 bis 20,0 Gew.-%, bevorzugt 0,05 bis 10 Gew.-%, besonders bevorzugt 0,1 bis 5 Gew.-%, bezogen auf das Gesamtgewicht des anwendungsbereiten Mittels, enthält.

7. Mittel nach einem der Ansprüche 1 bis 6, **dadurch gekennzeichnet, dass** das Mittel zusätzlich mindestens einen anorganischen Elektrolyt, bevorzugt Natriumchlorid, in einem Anteil von 0,1 bis 20 Gew.-%, bevorzugt 0,5 bis 5 Gew.-%, bezogen auf das Gesamtgewicht des anwendungsbereiten Mittels, enthält.

8. Mittel nach einem der Ansprüche 1 bis 7, **dadurch gekennzeichnet, dass** das Mittel eine Viskosität von 1000 bis 30000 mPa·s, bevorzugt von 3000 bis 10000 mPa·s, jeweils gemessen mit Haake Messgerät VT 550 bei 20 °C, einer Rotations-Frequenz von 8 min⁻¹ und mit einem Dreh-/Messsystem MV II, besitzt.

9. Verfahren zu Behandlung menschlicher Haare, **dadurch gekennzeichnet, dass** ein Mittel nach einem der Ansprüche 1 bis 8 auf menschliches Haar aufgetragen wird, für eine Einwirkdauer von 5 bis 45 min im Har belassen wird und anschließend das Haar mit einer wässrigen Zubereitung ausgespült wird.

10. Verwendung eines Mittels gemäß einem der Ansprüche 1 bis 8 zur Neutralisierung von gelblichen Verfärbungen auf ergrauten menschlichen Haaren.
